# EUROPÄISCHE PATENTSCHRIFT

(11) **EP 1 513 654 B1**
(45) Veröffentlichungstag und Bekanntmachung des Hinweises auf die Patenterteilung: **08.08.2007**
(21) Anmeldenummer: 02732310.4
(22) Anmeldetag: 13.06.2002
(51) Int. Cl.: B25B 23/10

(54) **SCHRAUBENDREHER MIT SCHRAUBENHALTER**
SCREW DRIVER WITH INTEGRATED SCREW HOLDER
TOURNEVIS MUNI D'UN PORTE-VIS

(43) Veröffentlichungstag der Anmeldung: 16.03.2005
(73) Patentinhaber: Synthes GmbH, 4436 Oberdorf (CH)
(72) Erfinder: NUSSBAUM, Fernand, CH-2544 Bettlach (CH); CICOIRA, Franco, CH-2545 Selzach (CH)
(74) Vertreter: Lusuardi, Werther
(86) Internationale Anmeldenummer: PCT/CH2002/000322
(87) Internationale Veröffentlichungsnummer: WO 2003/106110

(56) Entgegenhaltungen:
- EP-A- 0 458 449
- DE-A- 1 775 287
- DE-C- 348 078
- FR-A- 981 278
- US-A- 4 060 114
- US-A1- 2001 022 120

## Beschreibung

Die Erfindung bezieht sich auf einen Schraubendreher mit Schraubenhalter, gemäss dem Oberbegriff des Patentanspruchs 1.

Damit während der chirurgischen Implantation eines Implantates, welches an einem Knochen, einem Knochenfragment oder an einem Gelenk am menschlichen oder tierischen Körper befestigt werden soll, die umliegenden Weichteile eine möglichst geringe Schädigung erfahren, soll der Knochen oder beispielsweise auch ein Wirbelsäulensegment ohne grossflächiges Freilegen der zu behandelnden Teile operiert werden (Minimal Invasive Technik). Bei sehr kleinen Öffnungen der Weichteile eignen sich dann beispielsweise Klemmzangen zum Einführung von Knochen- oder Pedikelschrauben nicht mehr.

Ein Schraubendreher mit Mitteln zum Festhalten einer Schraube ist aus der EP 0 458 449 RYDER bekannt. Dieser bekannte Schraubendreher umfasst einen longitudinalen Schaftteil mit einem freien Ende, welches in eine entsprechende Vertiefung in einem Schraubenkopf einführbar ist. Die elastischen Mittel, welche zum Festklemmen der Schrauben dienen, sind in einer zur Längsachse parallelen Nute eingelassen und bestehen aus einem komprimierbaren Elastomer. Da die elastischen Mittel im unkomprimierten Zustand radial über mindestens eine Seitenfläche des Schaftteiles vorstehen, werden diese beim Einstecken des Schaftteiles in eine komplementäre Vertiefung komprimiert und drücken gegen die Seitenwand der Vertiefung, wodurch der Schraubenkopf am Schaftteil festgehalten wird. Nachteilig an dieser Ausgestaltung der elastischen Mittel ist, dass die Bohrung zur Aufnahme der elastischen Mittel relativ kompliziert ausgestaltet und daher der Schraubendreher für sehr kleine Schrauben ungeeignet ist.

Hier will die Erfindung Abhilfe schaffen. Der Erfindung liegt die Aufgabe zugrunde, einen Schraubendreher mit einem elastischen Schraubenhalter zu schaffen, welcher auch für kleinste Schrauben einsetzbar ist.

Die Erfindung löst die gestellte Aufgabe mit einem Schraubendreher mit einem Schraubenhalter, welcher die Merkmale des Anspruchs 1 aufweist, sowie mit einem Federelement zur Verwendung als Schraubenhalter an einem Schraubendreher, welches die Merkmale des Anspruchs 15 aufweist.

Der erfindungsgemässe Schraubendreher mit Schraubenhalter umfasst im wesentlichen einen Schaft und ein quer zur Längsachse angeordnetes Federelement. Der Schaft ist an seinem hinteren Schaftabschnitt mit Antriebsmitteln, beispielsweise einem Handgriff oder einer Maschine verbindbar und umfasst ein vorderes Schaftsegment, welches in eine für Schraubendreher geeignete Aufnahme, beispielsweise einen Innensechskant, Innensechsrund, Kreuzschlitz oder Torx an einem Schraubenkopf einführbar ist. Das Federelement ist beim vorderen Ende des Schaftes in eine Vertiefung mit quer zur Längsachse gerichteter Längsachse eingefügt. Der Querschnitt des vorderen Schaftsegmentes ist in den verschiedenen Ausführungsformen der erfindungsgemässen Vorrichtung polygon- oder sternförmig ausgebildet, wobei die Ecken oder Spitzen eckig oder abgerundet sein können und die Seiten gerade oder konkav ausgestaltet sein können. Die Längsachse der Vertiefung für das Federelement fällt mit einem der Radien, auf welchen die Ecken oder Spitzen liegen zusammen. Ferner ist das Federelement mindestens auf seinem über den Querschnitt hinausragenden Teil in der zur Längsachse des Schaftes orthogonalen Querschnittsfläche betrachtet quer zu Längsachse deformierbar.

Die durch die Erfindung erreichten Vorteile sind im wesentlichen darin zu sehen, dass dank der erfindungsgemässen Ausgestaltung des Federelementes:
- eine geringe Bautiefe des vorderen Schaftsegmentes erreichbar ist, so dass Schrauben mit geringer Höhe des Schraubenkopfes oder mit geringer Tiefe der zur Aufnahme des Schraubendrehers dienenden Vertiefungen einsetzbar sind;
- das vordere Schaftsegment kann prismatisch oder zylindrisch ausgestaltet werden, wodurch im Vergleich zu konischen Schaftsegmenten eine maximale Kraftübertragung auf die Schraube ausübbar ist (Vermeidung des sog. "cam out" Effektes;
- das Federelement auch nachträglich an handelsüblichen Schraubendrehern montierbar ist;
- keine Schraubenhaltehülse notwendig ist, wodurch eine einhändige Bedienung des Schraubendrehers möglich ist;
- eine einfache Herstellung und somit ein geringer Preis der Vorrichtung möglich ist; und
- das Federelement wegen der Notwendigkeit einer einzigen, radialen Bohrung im vorderen Schaftsegment auch für kleinste Schrauben einsetzbar ist und somit auch für Schrauben in der Maxillofacial-, Hand- und Fusschirurgie verwendbar ist.

In der bevorzugten Ausführungsform des erfindungsgemässen Schraubendrehers ist die Vertiefung als Bohrung mit kreiszylindrischer Form ausgestaltet, so dass die Vertiefung ohne grossen Aufwand auch nachträglich an handelsüblichen Schraubendrehern angebracht werden kann und ein Federelement einsetzbar ist.

In einer anderen Ausführungsform des erfindungsgemässen Schraubendrehers sind die Vertiefung und das Federelement mit prismatischer Form ausgestaltet. Damit ist der Vorteil erreichbar, dass sich das Federelement in der Vertiefung nicht verdrehen kann.

In wiederum einer anderen Ausführungsform des erfindungsgemässen Schraubendrehers ist das Federelement aus einem metallischen Werkstoff, vorzugsweise aus rostfreiem Federstahl oder superelastischem Metall, beispielsweise Nitinol gefertigt, so dass es für medizinische Anwendungszwecke geeignet ist und zudem eine lange Lebensdauer aufweist.

In einer weiteren Ausführungsform des erfindungsgemässen Schraubendrehers ist das Federelement aus einem Kunststoff, vorzugsweise aus einem Thermoplast (z.B. POM) gefertigt. Die Vorteile eines aus Kunststoff gefertigten Federelementes liegen in der rationelleren Fertigung (z.B. Spritzgiessen), den geringeren Kosten und der Elimination der Korrosionsgefahr.

In wiederum einer weiteren Ausführungsform des erfindungsgemässen Schraubendrehers weist das Federelement an seinem zweiten, in der Aufnahme in einem Schraubenkopf zur Anlage bringbaren Ende einen offenen Schlitz auf. Dieser Schlitz ist bezüglich des zur Längsachse der Vertiefung orthogonalen Federelementquerschnittes diametral angeordnet und teilt das Federelement beim zweiten Ende in zwei Zungen, welche quer zur Längsachse der Vertiefung und quer zur Längsachse des Schaftes elastisch bewegbar sind. Diese Ausführung des Federelementes gestattet es, eine für die chirurgische Anwendung notwendige, maximale Festhaltekraft für Schrauben zu erreichen, so dass die Gefahr, dass die Schrauben während des chirurgischen Eingriffes im menschlichen Körper verloren gehen, minimiert werden kann. Vorzugsweise betragen die Abmessungen
- Durchmesser des Federelementes zwischen 0,3 mm und 2,0 mm;
- Breite des Schlitzes zwischen 0,1 mm und 1,5 mm; und
- Länge des Schlitzes zwischen 0,5 mm und 3,0 mm.

Typischerweise betragen die Abmessungen:
- Durchmesser des Federelementes zwischen 0,5 mm und 1,5 mm;
- Breite des Schlitzes zwischen 0,2 mm und 1,2 mm; und
- Länge des Schlitzes zwischen 0,8 mm und 2,0 mm.

Ferner kann der Schlitz nach aussen, d.h. gegen das über der Querschnitt des vorderen Schaftsegmentes hinausragende Ende des Federelementes keilförmig erweitert sein, wobei der Keilwinkel vorzugsweise zwischen 0,1° und 10° beträgt, wodurch der Vorteil erreichbar ist, dass die Spannungsverteilung im Federelement optimiert wird. Dadurch ergeben sich bessere Federungseigenschaften und die Gefahr eines Ermüdungsbruches wird verringert.

In einer anderen Ausführungsform des erfindungsgemässen Schraubendrehers ist das zweite, über den Querschnitt des vorderen Schaftsegmentes vorstehende Ende des Federelementes mit einer Verjüngung ausgestattet, so dass die radial elastisch deformierbaren Zungen beim Einschieben des vorderen Schaftsegmentes in eine entsprechende Aufnahme für einen Schraubendreher in einem Schraubenkopf einfacher quer zur Längsachse des Schaftes komprimierbar sind. Die Verjüngung kann in Form einer Anschrägung, einer Abrundung, einer Anfasung oder einer runde Kante vorliegen.

Vorzugsweise beträgt das Verhältnis zwischen der parallel zur Längsachse des Federelementes gemessenen Länge der Verjüngung und dem Durchmesser des Federelementes zwischen 5 % und 30 %. Anstelle einer Verjüngung ist auch eine konvexe Ausbildung des zweiten Endes des Federelementes möglich.

In wiederum einer anderen Ausführungsform des erfindungsgemässen Schraubendrehers umfasst dieser mehrere Federelemente, welche in mehreren Vertiefungen am vorderen Schaftsegment einsetzbar sind. Die Längsachsen der Vertiefungen liegen vorzugsweise jeweils auf einem der die Ecken oder Rundung schneidenden Radien der zur Längsachse des Schaftes orthogonalen Querschnittsfläche des vorderen Schaftsegmentes. Durch den Einsatz mehrerer Federelemente lässt sich die auf den Schraubenkopf ausübbare Haltekraft für die Schraube am Schraubendreher erheblich steigern.

In einer weiteren Ausführungsform des erfindungsgemässen Schraubendrehers weist die Vertiefung von der Ecke oder Spitze gemessen in einer Tiefe T einen Absatz auf, wobei die Tiefe T gegenüber der zur Längsachse des Federelementes parallelen Länge des Federelementes gleich oder grösser ist, so dass das Federelement nicht über die Spitze, Ecke oder Abrundung des vorderen Schaftsegmentes hinausragt. Dadurch lässt sich vermeiden, dass das Federelement beim Einfügen des vorderen Schaftsegmentes in die Aufnahme für den Schraubendreher am Schraubenkopf nicht weiter in die Vertiefung hineingeschoben wird.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1a einen Längsschnitt durch eine Ausführungsform des erfindungsgemässen Schraubendrehers mit Schraubenhalter;
Fig. 1b einen Querschnitt durch die in Fig. 1a dargestellten Ausführungsform des erfindungsgemässen Schraubendrehers mit Schraubenhalter;
Fig. 2 einen Querschnitt durch eine andere Ausführungsform des erfindungsgemässen Schraubendrehers mit Schraubenhalter;
Fig. 3 einen Querschnitt durch die in den Fig. 1a und 1b dargestellte Ausführungsform des erfindungsgemässen Schraubendrehers mit in eine Vertiefung zur Aufnahme von Schraubendrehern an einem Schraubenkopf eingeführtem vorderen Schaftsegment; und
Fig. 4 eine Seitenansicht einer Ausführungsform eines Federelementes.

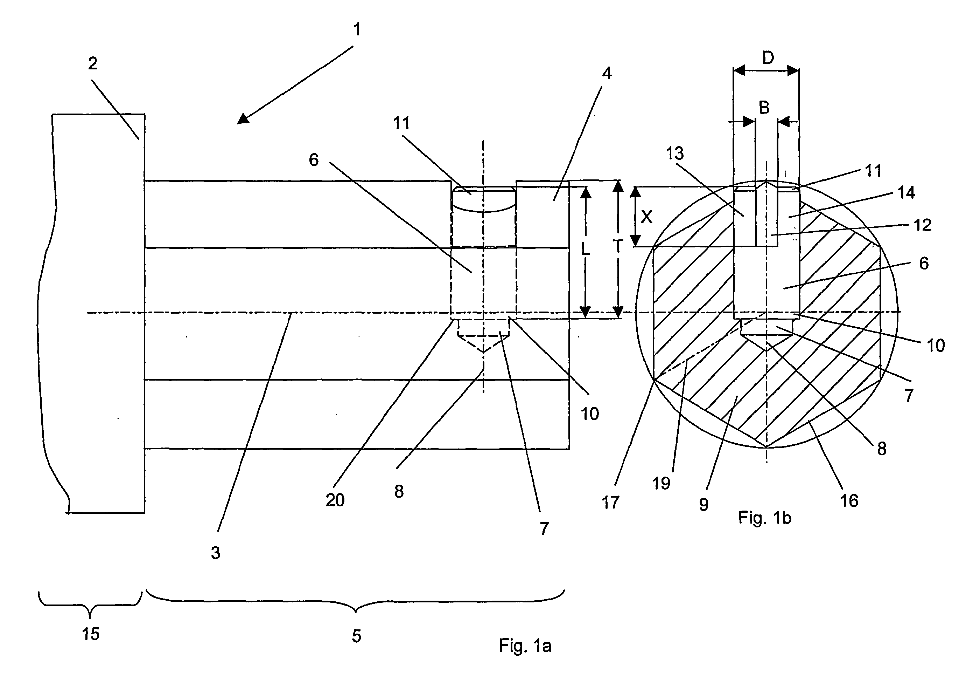

Der in den Fig. 1a und 1b dargestellte Schraubendreher 1 mit Schraubenhalter umfasst im wesentlichen einen longitudinalen Schaft 2 mit einer Längsachse 3, einem hinteren Schaftsegment 15 und einem vorderen Schaftsegment 5, und ein Federelement 6 mit einem Durchmesser D. Das vordere Schaftsegment 5 weist eine sechseckige, zur Längsachse 3 orthogonale Querschnittsfläche 9 mit geraden Seiten 16 und Ecken 17 auf und ist in eine für Schraubendreher geeignete Aufnahme an einem Schraubenkopf einführbar. Das Federelement 6 ist am vorderen Schaftsegment 5 in eine longitudinale Vertiefung 7 in Form einer kreiszylindrischen Bohrung mit senkrecht zur Längsachse 3 stehender Längsachse 8 eingepresst. Die sechs Ecken 17 der Querschnittsfläche 9 liegen auf sechs Radien 19, wobei die Längsachse 8 mit einem der Radien 19 zusammenfällt. Das Federelement 6 steht neben den Ecken 17 über die angrenzenden Seiten 16 vor. Damit des Federelement 6 beim Einführen des vorderen Schaftsegmentes 5 in die zur Aufnahme eines Schraubendrehers dienende Aufnahme an einem Schraubenkopf nicht in die Vertiefung 7 hineingedrückt wird, ist in der Vertiefung 7 ein axialer Absatz 20 angebracht, worauf das erste Ende 10 des Federelementes 6 aufsteht.

Ferner umfasst das Federelement 6 einen vom zweiten Ende 11 des Federelementes 6 in dieses eindringenden Schlitz 12 mit einer Breite B und einer Länge X. Der Schlitz 12 durchdringt das Federelement 6 diametral und ist parallel zur Längsachse 8 der Vertiefung 7 angeordnet. Das zweite Ende 11 des Federelementes 6 wird durch den Schlitz 12 in zwei parallel zur Längsachse 8 stehende Zungen 13;14 unterteilt, welche quer zur Längsachse 8 und gegen den Rand der Querschnittsfläche 9 hin elastisch deformierbar, d.h. gegen die Längsachse 8 hin einfederbar sind. Die Länge L des Federelementes 6 ist hier kleiner als die Tiefe T, welche von der Ecke 17 bis zum Absatz 20 reicht, so dass das Federelement 6 an der Ecke 17 gegenüber dem Querschnitt 9 vertieft ist.

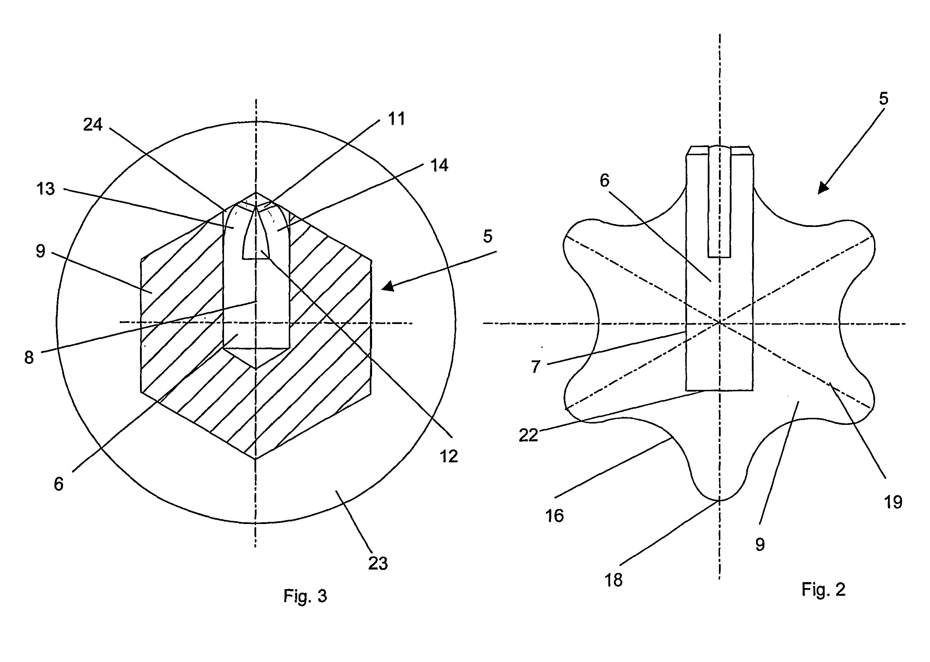

Die in Fig. 2 dargestellte Ausführungsform des erfindungsgemässen Schraubendrehers 1 unterscheidet sich von der in den Fig. 1a und 1b dargestellten Ausführungsform lediglich darin, dass der Querschnitt 9 des vorderen Schaftsegmentes 5 sternförmig ausgestaltet ist. Die auf den Radien 19 liegenden Spitzen 18 des Querschnittes 9 sind konvex abgerundet und die zwischen den Spitzen 18 liegenden Seiten 16 sind konkav gerundet. Daneben steht das Federelement 6 auf dem Grund 22 der Bohrung 7 auf.

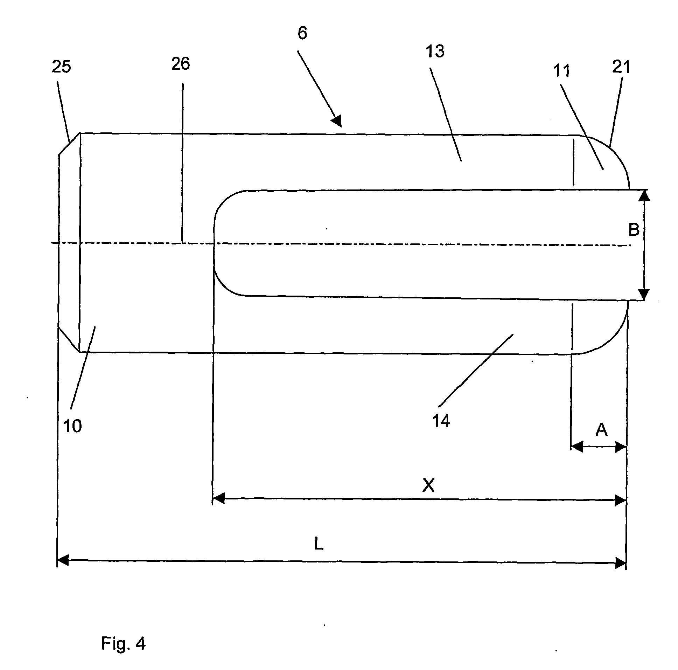

Fig. 3 zeigt das vordere Schaftsegment 5 gemäss der in den Fig. 1a und 1b dargestellten Ausführungsform des erfindungsgemässen Schraubendrehers, wenn es in eine Aufnahme 24 für Schraubendreher an einem Schraubenkopf 23 eingeführt ist. Das zweite Ende 11 des Federelementes 6 ist dann komplett im Querschnitt 9 eingeschlossen. Die beiden elastisch deformierbaren Zungen 13;14 werden gegen die Längsachse 8 gedrückt. Damit die beiden Zungen 13;14 ausreichend deformierbar sind und sich bei der Längsachse 8 nicht berühren, müssen die Tiefe T der Vertiefung 7 (Fig. 1a) sowie die Abmessungen des Federelementes 6, d.h der Durchmesser D und die Länge L und die Breite B des Schlitzes 12 (Fig. 1a) so aufeinander abgestimmt sein, dass einerseits die Deformation der Zungen 13;14 eine genügend hohe Klemmkraft auf die Seitenwände der Aufnahme 24 bewirkt und andererseits die Zungen 13;14 die Deformation nicht behindern.

In Fig. 4 ist eine Ausführungsform des erfindungsgemässen Federelementes 6 zur Verwendung als Schraubenhalter an einem Schraubendreher dargestellt. Das Federelement 6 ist kreiszylindrisch ausgestaltet, weist eine Längsachse 26 auf und umfasst an seinem ersten Ende 10 eine Anfasung 25 sowie eine konvex gerundete Verjüngung 21 an seinem zweiten Ende 11, welche parallel zur Längsachse 26 gemessen eine Länge A aufweist. In der hier dargestellten Ausführungsform beträgt das Verhältnis A/D zwischen der Länge A dieser Verjüngung 21 und dem Durchmesser D des Federelementes 6 25%, während das Verhältnis X/L zwischen der Tiefe X des Schlitzes 12 und der Länge L des Federelementes 6 75% beträgt. Der Schlitz 12 weist am vorderen Ende 11 des Federelementes 6 eine Breite B auf und erweitert sich gegen das vordere Ende 11 des Federelementes 6 mit einem Keilwinkel von 2°. Das Verhältnis B/D zwischen der Breite B und dem Durchmesser D beträgt hier 50%. Die beiden Zungen 13;14 sind senkrecht zur Längsachse 8 der Vertiefung 7 (Fig. 1a) elastisch deformierbar ausgestaltet, wobei die Federkräfte durch den Durchmesser D des Federelemtes 6 und die Abmessungen des Schlitzes 12 bestimmt sind.

## Patentansprüche

1. Schraubendreher mit Schraubenhalter, umfassend
A) einen mit Antriebsmitteln verbindbaren Schaft (2) mit einer Längsachse (3), einer zur Längsachse (3) orthogonalen Querschnittsfläche (9), einem vorderen Ende (4) und einem vorderen Schaftsegment (5), welches in eine für Schraubendreher geeignete Aufnahme an einem Schraubenkopf einführbar ist und in welchem mindestens eine longitudinale Vertiefung (7) mit einer quer zur Längsachse (3) gerichteten Längsachse (8) angebracht ist; und
B) mindestens ein in der mindestens einen Vertiefung (7) eingefügtes, quer zur Längsachse (3) über die Querschnittsfläche (9) des vorderen Schaftsegmentes (5) hinausragendes Federelement (6), wobei
C) die Querschnittsfläche (9) polygon- oder sternförmig mit geraden oder gekrümmten Seiten (16) und kantigen oder gerundeten, auf Radien (19) des Umkreises der Querschnittsfläche (9) des vorderen Schaftsegments (5) liegenden Ecken (17) oder Spitzen (18) ausgebildet ist
**dadurch gekennzeichnet, dass**
D) die Längsachse (8) der Vertiefung (7) mit einem der Radien (19) zusammenfällt; und
E) das Federelement (6) auf seinem über die Aussenkontur des vorderen Endes (4) hinausragenden Teil in der zur Längsachse (3) orthogonalen Querschnittsfläche (9) betrachtet quer zur Längsachse (8) der Vertiefung (7) in der Querschnittsflächenebene des vorderen Schaftsegments (5) elastisch deformierbar ist.

2. Schraubendreher nach Anspruch 1 , **dadurch gekennzeichnet, dass** die Vertiefung (7) eine Bohrung ist, vorzugsweise mit kreiszylindrischer Form.

3. Schraubendreher nach Anspruch 1 , **dadurch gekennzeichnet, dass** die Vertiefung (7) ein prismatischer Hohlraum ist.

4. Schraubendreher nach einem der Ansprüche 1 bis 3, **dadurch gekennzeichnet, dass** das Federelement (6) ein in der Vertiefung (7) liegendes, erstes Ende (10), ein über die Querschnittsfläche (9) hinausragendes, zweites Ende (11) und einen vom zweiten Ende (11) in das Federelement (6) eindringenden, zur Vertiefung (7) diametralen und zur Längsachse (3) parallelen Schlitz (12) umfasst, so dass durch den Schlitz (12) am zweiten Ende (11) des Federelementes (6) zwei bezüglich der Längsachse (8) der Vertiefung (7) radial elastisch deformierbare Zungen (13;14) gebildet werden.

5. Schraubendreher nach einem der Ansprüche 1 bis 4, **dadurch gekennzeichnet, dass** das Federelement (6) aus einem Metall, vorzugsweise aus rostfreiem Federstahl oder superelastischem Metall, beispielsweise Nitinol gefertigt ist.

6. Schraubendreher nach einem der Ansprüche 1 bis 4, **dadurch gekennzeichnet, dass** das Federelement (6) aus einem Kunststoff, vorzugsweise aus einem Thermoplast, beispielsweise POM gefertigt ist.

7. Schraubendreher nach einem der Ansprüche 1 bis 6, **dadurch gekennzeichnet, dass** sich der Schlitz (12) gegen das zweite Ende (11) him keilförmig erweitert, vorzugsweise mit einem Keilwinkel von 0,1° bis 10,0°.

8. Schraubendreher nach einem der Ansprüche 1 bis 7, **dadurch gekennzeichnet, dass** das erste Ende (10) des Federelementes (6) eine Verjüngung (21) gegen das zweite Ende (11) hin aufweist.

9. Schraubendreher nach Anspruch 8, **dadurch gekennzeichnet, dass** die Verjüngung (21) in Form einer Anschrägung, einer Abrundung, einer Anfasung oder einer runde Kante vorliegt.

10. Schraubendreher nach Anspruch 9; **dadurch gekennzeichnet, dass** das Federelement (6) einen Durchmesser D aufweist und das Verhältnis der parallel zur Längsachse (8) der Vertiefung (7) gemessenen Länge A der Verjüngung (21) und dem Durchmesser D zwischen 5 % und 30 % beträgt.

11. Schraubendreher nach einem der Ansprüche 1 bis 10, **dadurch gekennzeichnet, dass** das zweite Ende (11) des Federelementes (6) endständig konvex ausgebildet ist.

12. Schraubendreher nach einem der Ansprüche 4 bis 11, **dadurch gekennzeichnet, dass** das Federelement (6) einen Durchmesser D zwischen 0,3 mm und 2 mm hat; und der Schlitz (12) eine Breite B zwischen 0,1 mm und 1,5 mm und eine Länge X zwischen 0,5 mm und 3 mm aufweist.

13. Schraubendreher nach einem der Ansprüche 1 bis 12, **dadurch gekennzeichnet, dass** sie mehrere Federelemente (6) umfasst, welche in Vertiefungen (7) einfügbar sind, deren Längsachsen (8) jeweils mit einem der Radien (19) zusammenfallen.

14. Schraubendreher nach einem der Ansprüche 1 bis 13, **dadurch gekennzeichnet, dass** die Vertiefung (7) von der Ecke (17) oder Spitze (18) gemessen in einer Tiefe T einen Absatz (20) aufweist; und das Federelement (6) parallel zur Längsachse (8) der Vertiefung (7) gemessen eine Länge L aufweist, wobei L ≤ T ist.

## Claims

1. Screwdriver with a screw holder, comprising
A) a shaft (2), which can be connected with driving means and has a longitudinal axis (3), a cross-sectional area (9) orthogonal to the longitudinal axis (3), a front end (4) and a front segment (5) of the shaft, which can be inserted into a seat at a screw head, suitable for the screwdriver, and in which there is at least one longitudinal recess (7) with a longitudinal axis (8), directed transversely to the longitudinal axis (3), and
B) at least one spring element (6), inserted into the at least one recess (7) and protruding transversely to the longitudinal axis (3) over the cross-sectional area (9) of the front segment (5) of the shaft,
C) the cross-sectional area (9) being polygonal or star-shaped with straight or curved sides (16) and angular or rounded corners or tips (18) lying on radii (19) of the circumcircle of the cross-sectional area (9) of the front segment (5) of the shaft,
**characterized in that**
D) the longitudinal axis (8) of the recess (7) coincides with one of the radii (19); and
E) the spring element (6) is - viewed in the cross-sectional area (9) orthogonal to the longitudinal axis (3) - elastically deformable transversely to the longitudinal axis (8) of the recess (7) in the cross-sectional plane of the front segment (5) of the shaft on its part protruding beyond the outer contour of the front end (4).

2. The screwdriver of claim 1, **characterized in that** the recess (7) is a borehole, preferably with a cylindrical shape.

3. The screwdriver of claim 1, **characterized in that** the recess (7) is a prismatic cavity.

4. The screwdriver of one the claims 1 to 3, **characterized in that** the spring element (6) comprises a first end (10), which lies in the recess (7), a second end (11), which protrudes beyond the cross-sectional area (9) and a slot (12), penetrating from the second end (11) into the spring element (6), and diametrically to the recess (7) and parallel to the longitudinal axis (3), so that to cogs (13; 14), which can be deformed radially and elastically with respect to the longitudinal axis (8) of the recess (7), are formed by the slot (12) at the second end (6).

5. The screwdriver of one of the claims 1 to 4, **characterized in that** the spring element (6) is made from a metal, preferably from stainless spring steel or from a superelastic metal, such as Nitinol.

6. The screwdriver of one the claims 1 to 4, **characterized in that** the spring element (6) is made from a plastic, preferably from a thermoplastic material, such as POM.

7. The screwdriver of one the claims 1 to 6, **characterized in that** the slot (12) expands in wedge-shaped fashion towards the second end (11), preferably at a wedge angle of 0.1 to 10.0°.

8. The screwdriver of one of the claims 1 to 7, **characterized in that** the first end (10) of the spring element (6) has a taper (21) in the direction of the second end (11).

9. The screwdriver of claim 8, **characterized in that** the taper (21) is in the form of an obliqueness, a curvature or a bevel.

10. The screwdriver of claim 9, **characterized in that** the spring element (6) has a diameter D and that the ratio of the length A of the taper (21), measured parallel to the longitudinal axis (8) of the recess (7), to the diameter D is between 1:20 and 3:10.

11. The screwdriver of one the claims 1 to 10, **characterized in that** the second end (11) of the spring element (6) is terminally convex.

12. The screwdriver of one the claims 4 to 11, **characterized in that** the spring element (6) has a diameter D of between 0.3 mm and 2 mm and the slot (12) has a width B of between 0.1 mm than 1.5 mm and a length X of between 0.5 mm and 3 mm.

13. The screwdriver of one of the claims 1 to 12, **characterized in that** it comprises several spring elements (6), which can be inserted in recesses (7) the longitudinal axes (8) of which coincide with one of the radii (19) each.

14. The screwdriver of one the claims 1 to 13, **characterized in that** the recess (7), at a depth T, measured from the corner (17) or the tip (18), has a ledge (20); and that the spring element (6) has a length L, measured parallel to the longitudinal axis (8) of the recess (7), with L ≤ T.

## Revendications

1. Tournevis avec porte-vis, comprenant
A) une queue (2) pouvant être reliée à des moyens d'entraînement, ayant un axe longitudinal (3), une section transversale (9) orthogonale à l'axe longitudinal (3), une extrémité avant (4) et un segment avant de queue (5) qui peut être inséré dans un logement approprié pour un tournevis sur une tête de vis et dans lequel au moins un évidement longitudinal (7) est prévu avec un axe longitudinal (8) transversal à l'axe longitudinal (3) ; et
B) au moins un élément élastique (6) inséré dans le au moins un évidement (7), dépassant, transversalement à l'axe longitudinal (3), de la section transversale (9) du segment avant de queue (5), dans lequel
C) la section transversale (9) est réalisée sous forme polygonale ou en étoile avec des côtés droits ou courbés (16) et des coins (17) ou sommets (18) angulaires ou arrondis, situés sur les rayons (19) de la circonférence de la section transversale (9) du segment avant de queue (5),
**caractérisé en ce que**
D) l'axe longitudinal (8) de l'évidement (7) coïncide avec l'un des rayons (19) ; et
E) l'élément élastique (6), vu au niveau de sa partie dépassant du contour externe de l'extrémité avant (4) dans la section transversale (9) orthogonale à l'axe longitudinal (3), peut être déformé de manière élastique transversalement à l'axe longitudinal (8) de l'évidement (7) dans le plan de la section transversale du segment avant de queue (5).

2. Tournevis selon la revendication 1, **caractérisé en ce que** l'évidement (7) est un alésage, de préférence en forme de cylindre circulaire.

3. Tournevis selon la revendication 1, **caractérisé en ce que** l'évidement (7) est un espace creux prismatique.

4. Tournevis selon l'une quelconque des revendications 1 à 3, **caractérisé en ce que** l'élément élastique (6) comprend une première extrémité (10) située dans l'évidement (7), une deuxième extrémité (11) dépassant de la section transversale (9) et une fente (12) rentrant dans l'élément élastique (6) par la deuxième extrémité (11), diamétralement opposée à l'évidement (7) et parallèle à l'axe longitudinal (3), de sorte que la fente (12) permet de former, au niveau de la deuxième extrémité (11) de l'élément élastique (6), deux languettes radialement déformables de manière élastique (13 ; 14) par rapport à l'axe longitudinal (8) de l'évidement (7).

5. Tournevis selon l'une quelconque des revendications 1 à 4, **caractérisé en ce que** l'élément élastique (6) est fait de métal, de préférence d'acier à ressort inoxydable ou de métal superélastique, par exemple de Nitinol.

6. Tournevis selon l'une quelconque des revendications 1 à 4, **caractérisé en ce que** l'élément élastique (6) est fait de matière plastique, de préférence d'une matière thermoplastique, par exemple de POM.

7. Tournevis selon l'une quelconque des revendications 1 à 6, **caractérisé en ce que** la fente (12) s'élargit de manière cunéiforme vers la deuxième extrémité (11), de préférence selon un angle de conicité de 0,1° à 10,0°.

8. Tournevis selon l'une quelconque des revendications 1 à 7, **caractérisé en ce que** la première extrémité (10) de l'élément élastique (6) présente un rétrécissement (21) vers la deuxième extrémité (11).

9. Tournevis selon la revendication 8, **caractérisé en ce que** le rétrécissement (21) se présente sous la forme d'une inclinaison, d'un arrondi, d'un biseau ou d'un bord arrondi.

10. Tournevis selon la revendication 9, **caractérisé en ce que** l'élément élastique (6) présente un diamètre D et le rapport de la longueur A du rétrécissement (21), mesurée parallèlement à l'axe longitudinal (8) de l'évidement (7), au diamètre D est compris entre 5% et 30%.

11. Tournevis selon l'une quelconque des revendications 1 à 10, **caractérisé en ce que** la deuxième extrémité (11) de l'élément élastique (6) est réalisée de manière convexe au niveau de son extrémité.

12. Tournevis selon l'une quelconque des revendications 4 à 11, **caractérisé en ce que** l'élément élastique (6) présente un diamètre D compris entre 0,3 mm et 2 mm ; et la fente (12) présente une largeur B comprise entre 0,1 mm et 1,5 mm et une longueur X comprise entre 0,5 mm et 3 mm.

13. Tournevis selon l'une quelconque des revendications 1 à 12, **caractérisé en ce qu'**il comprend plusieurs éléments élastiques (6) qui peuvent être insérés dans des évidements (7) dont les axes longitudinaux (8) coïncident chacun avec l'un des rayons (19).

14. Tournevis selon l'une quelconque des revendications 1 à 13, **caractérisé en ce que** l'évidement (7), mesuré depuis le coin (17) ou le sommet (18), présente une butée (20) à une profondeur T ; et **en ce que** l'élément élastique (6) présente une longueur L mesurée parallèlement à l'axe longitudinal (8) de l'évidement (7), où L ≤ T.
